Europäisches Patentamt

European Patent Office

Office européen des brevets

(11) Publication number: **0 226 258 B1**

(12) **EUROPEAN PATENT SPECIFICATION**

(45) Date of publication of patent specification: **11.03.92**  (51) Int. Cl.[5]: **C01B 33/20**

(21) Application number: **86202271.2**

(22) Date of filing: **16.12.86**

(54) **Synthetic, crystalline, porous material containing silicon oxide, titanium oxide and iron oxide.**

(30) Priority: **19.12.85 IT 2329285**

(43) Date of publication of application:
**24.06.87 Bulletin 87/26**

(45) Publication of the grant of the patent:
**11.03.92 Bulletin 92/11**

(84) Designated Contracting States:
**AT BE CH DE ES FR GB GR LI LU NL SE**

(56) References cited:
**FR-A- 2 403 975**
**FR-A- 2 471 950**

**CHEMICAL ABSTRACTS, vol. 103, no. 16, 21st October 1985, page 424, abstract no. 129920z, Columbus, Ohio, US; JP-A-60 65 714 (AGENCY OF INDUSTRIAL SCIENCES AND TECHNOLOGY) 15-4-1985**

(73) Proprietor: **ENIRICERCHE S.p.A.**
**Corso Venezia 16**
**I-20121 Milan(IT)**

Proprietor: **ENICHEM SYNTHESIS S.p.A.**
**Via Ruggero Settimo 55**
**I-90139 Palermo(IT)**

Proprietor: **SNAMPROGETTI S.p.A.**
**Corso Venezia 16**
**I-20121 Milan(IT)**

(72) Inventor: **Bellussi, Giuseppe**
**Via A. Scopo 44**
**I-29100 Piacenza(IT)**
Inventor: **Clerici, Mario Gabriele**
**Via Europa 34**
**I-20097 San Donato Milanese Milan(IT)**
Inventor: **Giusti, Aldo**
**Via Di Meassino 390**
**I-55100 Lucca(IT)**
Inventor: **Buonomo, Franco**
**Via Trento 4**
**I-20097 San Donato Milanese Milan(IT)**

(74) Representative: **Roggero, Sergio et al**
**Ing. Barzanò & Zanardo Milano S.p.A. Via Borgonuovo 10**
**I-20121 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a synthetic material containing silicon oxide, titanium oxide and iron oxide, having a crystalline, porous, structure of zeolitic character and to the process for the preparation of such a material.

Such a material is structurally similar to ZSM-5 zeolite as disclosed in US-A-3,702,886, formally constituted, in its calcined and anhydrous form, by $M_{2/n}O, SiO_2, Al_2O_3$ (with M = cation with $n$ valency).

Other synthetic materials, structurally correlated to ZSM-5 zeolite are known, such as the one as disclosed in US-A-4,061,724, formally constituted, in its calcined and anhydrous form, by $SiO_2$ the one as disclosed in BE-886,812, formally constituted, in its calcined and anhydrous form, by $SiO_2$ and $TiO_2$ and the one as disclosed in FR-A-2,403,975 patent application, formally constituted, in its calcined and anhydrous form, by $M_{2/n}O$, $SiO_2$, $Fe_2O_3$ (with M = cation of n valency).

A novel synthetic zeolite has been found now, which we'll denominate "titanium-iron-silicalite", structurally similar to silicalite, which can be used either as molecular sieve or as ion exchanger, or as catalyst in the following reactions: cracking, selectoforming, hydrogenations and dehydrogenations, oligomerizations, alkylations, isomerizations, dehydrations of oxygen-containing organic compounds - selective hydroxylations of organic substrates by $H_2O_2$ (oxidation of olefins, hydroxylations of aromatics).

The synthetic, crystalline, porous material of zeolitic character, containing silicon, titanium and iron oxides, which is the object of the present invention, corresponds, in its calcined and anhydrous state, to the following empirical formula:

$$p\ HFeO_2\ .\ q\ TiO_2\ .\ SiO_2$$

wherein $p$ has a value higher than zero and lower than or equal to 0,050 and $q$ has a value higher than zero and lower than or equal to 0,025,
and the $H^+$ of $HFeO_2$ can be at least partly replaceable or replaced by cations.

The passage from a cationic form to another cationic form can be performed by any customary exchange processes of the known art.

The synthetic material in accordance with the present invention results crystalline on X-ray examination.

Such an examination has been carried out by means of a powder diffractometer, equipped with an electronic impulse counter system, by using $CuK-\alpha$ radiation. For the computation of the intensity values, the peak heights have been measured, and their percent heights relatively to the most intense peak have been computed.

The main reflections for the calcined and anhydrous product are characterized by the following $d$ values (wherein $d$ is the interplanar distance):

| d nm | $\underline{d}$ (Å) | Relative Intensity |
|---|---|---|
| 1,114 ± 0,010 | (11,14 ± 0,10) | vs |
| 0,999 ± 0,010 | (9,99 ± 0,10) | s |
| 0,974 ± 0,010 | (9,74 ± 0,10) | m |
| 0,636 ± 0,007 | (6,36 ± 0,07) | mw |
| 0,599 ± 0,007 | (5,99 ± 0,07) | mw |
| 0,426 ± 0,005 | (4,26 ± 0,05) | mw |
| 0,386 ± 0,004 | (3,86 ± 0,04) | s |
| 0,382 ± 0,004 | (3,82 ± 0,04) | s |
| 0,375 ± 0,004 | (3,75 ± 0,04) | s |
| 0,372 ± 0,004 | (3,72 ± 0,04) | s |
| 0,365 ± 0,004 | (3,65 ± 0,04) | m |
| 0,365 ± 0,002 | (3,05 ± 0,02) | mw |
| 0,299 ± 0,002 | (2,99 ± 0,02) | mw |

(wherein: vs = very strong; s = strong; m = medium; mw = medium-weak).

Such a diffraction spectrum is essentially similar to that of ZSM-5 and consequently of the other zeolites structurally correlated to ZSM-5, which have been mentioned in the introduction.

The material disclosed herein shows an I.R, spectrum characterized by the following most representative values of wn (wherein "wn" is the wave number):

| wn cm$^{-1}$ | Relative Intensity |
|---|---|
| 1220 - 1230 | w |
| 1080 - 1110 | s |
| 965 - 975 | mw |
| 795 - 805 | mw |
| 550 - 560 | m |
| 450 - 470 | ms |

(wherein: s = strong; ms = medium-strong; m = medium; mw = medium-weak; w = weak).

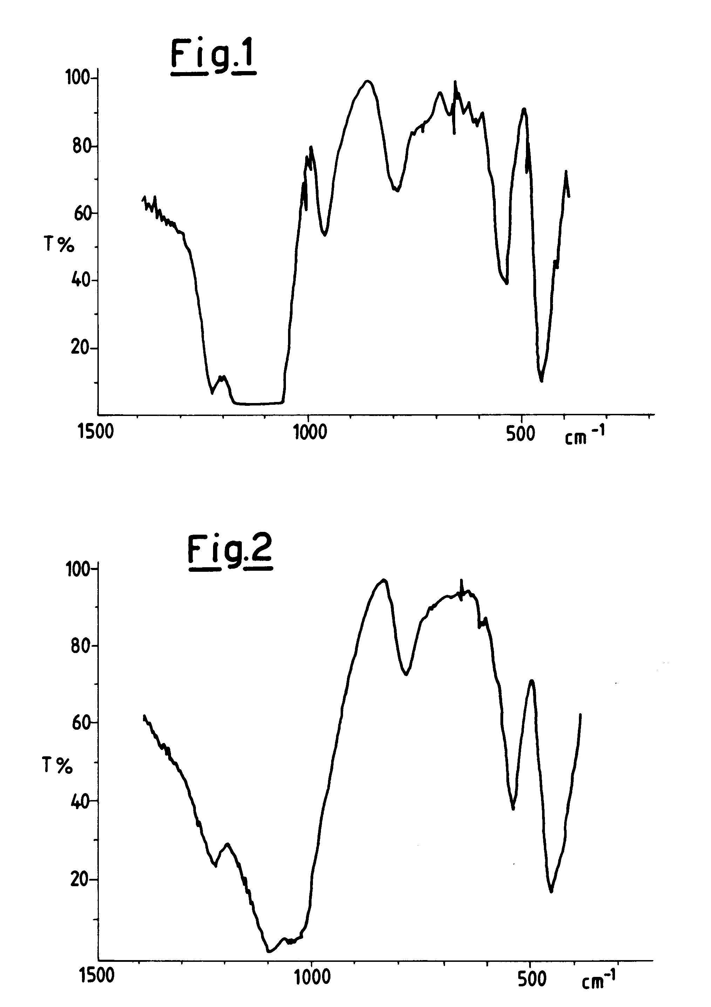

In Figure 1 the I.R. spectrum is shown, wherein on the abscissae the wave number in cm$^{-1}$ and on the ordinates the percent transmittance are expressed.

Such an I.R. spectrum is essentially similar to that of the zeolite as disclosed in BE-A-886,812, whilst it is considerably different from that of ZSM-5 (or from similar structures) and from that of the ferrosilicate as disclosed in FR-A-2,403,975, which have the I.R. spectrum as shown in Figure 2. It can be observed that in the spectrum of Fig. 2, the band at 965-975 cm$^{-1}$, which is characteristic of titanium silicalite of BE-886,812, and of titanium-iron-silicalite, does not appear.

Summarizing, the material as disclosed herein is different from ZSM-5 of US-A-3,702,886 and from the ferrosilicate as of FR-A-2,403,975, as to both its empirical formula and its I.R. spectrum; and relatively to the zeolite of BE-886,812 patent as to its empirical formula.

Furthermore, the use of the subject material of the present invention as a catalyst in the above listed reactions is a further confirmation of the difference of our product relatively to those of the prior art.

In fact, both ZSM-5 of US-A-3,702,886, and the ferrosilicate of FR-A-2,403,975 can be used as catalysts in such reactions as dehydrations of oxygenated organic compounds, cracking, selectoforming, hydrogenations and dehydrogenations, oligomerizations, alkylations, isomerizations, but they result inactive in the reactions between organic substrates and $H_2O_2$, (hydroxylation of phenol to diphenols, oxidation of olefins), whilst the zeolite of BE-A-886,812 results inactive for the first reactions and active for the last ones; to the contrary, our zeolite is active for all of the above mentioned reactions.

A second object of the present invention is the preparation process for the obtainment of the synthetic, crystalline, porous material as defined above.

Said process is characterized in that under hydrothermal conditions a derivative of silicon, a derivative of titanium, a derivative of iron and a nitrogenous organic base are reacted, with an $SiO_2/Fe_2O_3$ molar ratio of the reactants greater than 50, preferably comprised within the range of from 150 to 600, an $SiO_2/TiO_2$ molar ratio of the reactants greater than 5, preferably comprised within the range of from 15 to 25, an $H_2O/SiO_2$ molar ratio of the reactants preferably comprised within the range of from 10 to 100, more preferably of from 30 to 50, possibly in the presence of one or more salts and/or hydroxides of alkali or alkali-earth metals, with an $M/SiO_2$ molar ratio (wherein M is the alkali and/or alkali-earth cation) of the reactants lower than 0.1, preferably lower than 0,08, or equal to zero.

In the empirical formula of the material, the iron has been indicated in the $HFeO_2$ form, to underline that the material is in the $H^+$ form. When speaking of the ratios between the various reactants, we use, for iron, the $Fe_2O_3$ form, which is the most usual.

The silicon derivative is selected from silica gel, silica sol and alkyl silicates, among which, preferably, tetraethyl silicate; the titanium derivative is selected from titanium salts, such as, e.g., its halides, and organic titanium derivatives, such as, e.g., alkyltitanates, preferably tetraethyl titanate; the iron derivative is selected from iron salts, such as, e.g., its halides or the nitrates, the hydroxides, and the organic derivatives, such as, e.g., the alkoxides.

The nitrogenous organic base can be an alkylammonium hydroxide, preferably tetrapropyl-ammonium hydroxide.

In case tetrapropylammonium hydroxide is used, the $TPA^+/SiO_2$ ratio (wherein TPA = tetrapropylammonium) of the reactants is comprised within the range of from 0,1 to 1, preferably from 0,2 to 0,4.

The reactants are reacted with each other by operating at a temperature of from 100°C to 200°C, preferably of from 160°C to 180°C, at a pH comprised within the range of from 9 to 14, preferably of from 10 to 12, and for a time period ranging from 1 hour to 5 days, preferably from 3 hours to 10 hours.

To the purpose of better illustrating the meaning of the present invention, some preparation and use examples are given.

Example 1

3

This example shows the preparation of titanium-iron-silicalite.

0,65 g of $Fe(NO_3)_3.9H_2O$ is dissolved in water and from the solution the hydroxide is precipitated by means of the addition on ammonium hydroxide. The precipitate is filtered and washed by being redispersed in cold water, and filtered until the filtrate turns to neutral. The damp hydroxide is then dissolved in 54 g of solution at 18,7% by weight of tetrapropyl-ammonium hydroxide.

In a separate vessel, 2,28 g of tetraethyl-orthotitanate is dissolved in 41,6 g of tetraethyl-silicate and this solution is added to the previously prepared one, with stirring.

The whole mass is heated at 50°C - 60°C, always with stirring, until a single-phase solution is obtained, then 100 ml of water is added.

The obtained solution is charged to an autoclave and is heated, under its autogenous pressure, at 170°C over 4 hours.

The discharged product is centrifuged and washed twice by re-dispersion and centrifuging; it is then dried 1 hour at 120°C, and is then calcined 4 hours at 550°C in the air.

The obtained product has an $SiO_2/Fe_2O_3$ molar ratio of 394, and an $SiO_2/TiO_2$ molar ratio of 48.

Examples 2-6

In Table 1 other titanium-iron-silicalites are shown, which have been prepared by the same modalities as disclosed in Example 1, but with the composition of the reactants being varied.

From the above-reported preparation examples, one can observe that the maximum amounts of titanium and iron which can be obtained in the end product are not independent from each other.

The minimum $SiO_2/TiO_2$ ratio which can be obtained is of about 44, and it can be only obtained if $SiO_2/Fe_2O_3$ ratio in the reactant mixture is higher than about 250 (Examples 1, 2, 3).

By increasing $SiO_2/Fe_2O_3$ ratio in the reactant mixture, a decrease of $SiO_2/TiO_2$ ratio and an increase of $SiO_2/Fe_2O_3$ occurs in the obtained product (Examples 5, 4, 1, 2, 3).

$SiO_2/TiO_2$ ratio in the obtained product continues to decrease until it reaches its minimum value around 44, which is reached when $SiO_2/Fe_2O_3$ in the reaction mixture is of from 250 to 600; further increases of $SiO_2/Fe_2O_3$ ratio in the reactant mixture cause only $SiO_2/Fe_2O_3$ to increase in the obtained product, whilst $SiO_2/TiO_2$ remains nearly constant (Example 3).

The addition of alkali metals to the reaction mixture favours the decrease of $SiO_2/Fe_2O_3$ ratio in the obtained product (Example 6) ratio in the obtained product.

Example 7

Always in Table 1, a product is shown, which does not have the same characteristics as of the preceding products.

It can be observed from said Example that, when the $SiO_2/Fe_2O_3$ ratio in the reactant mixture is 30, in the absence of alkali metals no crystallization of the mixture occurs.

Example 8

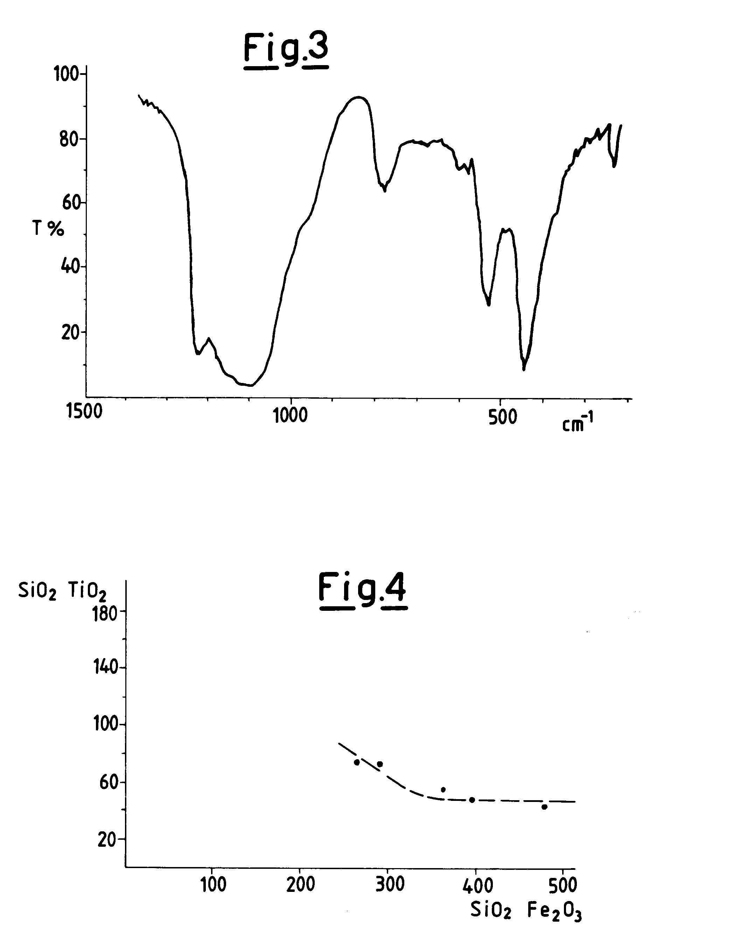

Always in Table 1, a product is shown, which has the I.R. spectrum of Fig. 3, from which it can be observed that the band at 965-975 $cm^{-1}$ is only sparingly shown, with a much lower intensity than that in the spectrum of Fig. 1, although the product of Example 8 has a higher titanium content than of Examples 1-3.

The addition of large amounts of alkali metals ($M^+/SiO^-_2 = 0,08$) in the reaction mixture can cause an increase in titanium amount, which can be detected from the chemical analysis of the obtained product, an $SiO_2/TiO_2$ ratio lower than 40 being obtained, but in such case $TiO_2$ is at least partly in a form different from the form it has in the titanium-iron-silicalite, and such as not to yield the spectrum of Fig. 1, but that of Fig. 3.

In Fig. 4, a chart is shown, which sets forth the dependence of the maximum amounts of titanium (shown in the ordinates as $SiO_2/TiO_2$, molar ratio) and of iron (shown in the abscissae as $SiO_2/Fe_2O_3$ molar ratio) which can be obtained in the end product.

Table 1

| Example | Reaction Mixture Composition | | | | | Crystallization time, hours | Crystallization Temperature, °C | Product Composition | |
|---|---|---|---|---|---|---|---|---|---|
| | $SiO_2/Fe_2O_3$ | $SiO_2/TiO_2$ | $Na^+/SiO_2$ | $TPA^+/SiO_2$ | $H_2O/SiO_2$ | | | $SiO_2/Fe_2O_3$ | $SiO_2/TiO_2$ |
| 1 | 250 | 20 | – | 0.25 | 40 | 4 | 170 | 394 | 48 |
| 2 | 600 | 20 | – | 0.25 | 40 | 4 | 170 | 474 | 43 |
| 3 | 1000 | 20 | – | 0.25 | 40 | 4 | 170 | 837 | 44 |
| 4 | 200 | 20 | – | 0.25 | 40 | 4 | 170 | 363 | 55 |
| 5 | 70 | 20 | – | 0.25 | 40 | 4 | 170 | 287 | 72 |
| 6 | 200 | 20 | 0.01 | 0.25 | 40 | 4 | 170 | 262 | 73 |
| 7 | 30 | 20 | – | 0.25 | 40 | 4 | 170 | no crystallization | |
| 8 | 70 | 20 | 0.08 | 0.25 | 40 | 15 | 170 | 54 | 23 |

Example 9

To a 1000 cm³ steel autoclave equipped with mechanical stirrer, temperature control system, pressure control system to operate under constant pressure, 70 g of water, 250 g of methanol, 4 g of catalyst

5

prepared according to Example 2 are charged.

To a drum connected with the autoclave, 54 g of 34% (weight/weight) $H_2O_2$ is charged. After that the temperature of the system has been set at the controlled value of 40°C, and being pressurized with propylene at the pressure of 5,884 bar (6 abs. atm). (constant throughout the test duration), the hydrogen peroxide is added, with strong stirring, to the suspension inside the autoclave.

The reaction is monitored by samples being drawn, at time intervals, and analysed. Hydrogen peroxide is determined to iodometric titration, and the reaction products are analysed by gas-chromatography.

After 1 hour the following situation has occurred:

$H_2O_2$ conversion : 95 %

Selectivity (relatively to $H_2O_2$),

propylene oxide : 80 %

1-methoxy-2-hydroxypropane : 11 %

2-methoxy-1-hydroxypropane : 5,5%

propylene glycol : 3,0%

Example 10

The process is carried out by means of the equipment and the modalities of Example 9. The reactants used are 420 g of $CH_3OH$, 4 g of catalyst (prepared according to Example 2) and 41 g of 34% (weight/weight) $H_2O_2$. The operating temperature is of 40°C and the propylene pressure is of 4 abs. atm. After 45 minutes of reaction, the situation of the system is as follows:

$H_2O_2$ conversion : 93 %

Selectivity (relatively to $H_2O_2$),

propylene oxide : 83 %

1-methoxy-2-hydroxypropane : 10 %

2-methoxy-1-hydroxypropane : 5,5%

propylene glycol : 1 %

Example 11

To a 1-l steel autoclave, equipped with mechanical stirrer and reaction temperature control system, 450 g of methanol 90 g of 1-octene, 4,5 g of catalyst (prepared according to Example 2) are charged.

To a drum connected with the autoclave, 45 g of 34% (weight/weight) $H_2O_2$ is charged. After that the temperature of the system has been set at the controlled value of 45°C, and with stirring, hydrogen peroxide is added to the other reactants. The reaction course is monitored by samples being drawn at regular time intervals. Hydrogen peroxide is determined by iodometric titration, and the reaction products are analysed by gas-chromatography.

After 1 hour, the situation is:

$H_2O_2$ conversion : 90 %

Octene conversion : 50,3%

Selectivity to 1,2-epoxyoctane : 78 %

Ethers + glycols : 21,5%

Example 12

The process is carried out by means of the same modalities and equipment as of Example 11.

To the autoclave, charged are 400 g of methanol, 90 g of allyl chloride, and 9 g of catalyst prepared according to Example 2; to the drum, 61 g of 34% (weight/weight) $H_2O_2$ is added. The reaction is carried out at the temperature of 60°C. Thirty minutes later, the situation is:

$H_2O_2$ conversion : 95 %

Allyl chloride conversion : 49 %

Epichlorohydrin selectivity (relatively to $H_2O_2$) : 80 %

Example 13

To a tubular steel reactor, 1.04 g is charged of catalyst of 18-40 mesh (1 - 0,42 mm) of granulometry, prepared according to Example 2. The reactor is placed inside and electrical oven and is gradually heated up to the reaction temperature, with a stream of dimethyl ether being flown through it. The gaseous reaction products are analyzed by in-line chromatography after the liquid products being condensed in a bath kept at 0°C - 5°C. These latter are separately weighed and analyzed, always by chromatographic way. The conversion and selectivity are computed according to the hereunder shown equations:

$$Conversion = \frac{(DME_{in} - DME_{out})}{(DME_{in})}$$

$$Selectivity = \frac{(mol\ of\ i\ product)}{(DME_{in} - DME_{out})}$$

(DME = dimethyl ether; in = incoming; out = outgoing).

The reaction conditions and the results obtained are gathered in Table 2.

Table 2

| | | | | |
|---|---|---|---|---|
| T | °C | | 360 | 380 |
| p | bar (atm) | | 0,98065[1] | 0,098065[1] |
| GHSV | (h$^{-1}$) | | 440 | 800 |
| Run hours | | | 3 | 3 |
| DME Conversion (%) | | | 86 | 84 |

Product Composition (% by weight - $H_2O$, DME excluded)

| | | |
|---|---|---|
| $CH_3OH$ | 11.0 | 67,2 |
| $CH_4$ | 0.4 | 0,5 |
| $C_2H_4$ | 0.7 | 0,7 |
| $C_2H_6$ | 0.01 | 0,01 |
| $C_3H_6$ | 8.6 | 18,6 |
| $C_3H_8$ | 0.1 | 0,3 |
| $\Sigma C_4$ | 2.7 | 9,6 |
| $\Sigma C_5$ | 0.3 | 1,9 |
| $\Sigma C_6^+$ | 76.1 | 1,1 |

**Claims**

**Claims for the following Contracting States : BE, CH, DE, FR, GB, GR, LI, LU, NL, SE**

**1.** Synthetic, crystalline, porous material of zeolitic character, containing silicon oxide, titanium oxide and iron oxide, characterized in that
it corresponds, in its calcined and anhydrous state, to the following empirical formula:

$$p \, HFeO_2 \cdot q \, TiO_2 \cdot SiO_2$$

wherein p has a value higher than zero and lower than or equal to 0,050 and q has a value higher than zero and lower than or equal to 0,025, the $H^+$ of $HFeO_2$ can be at least partly replaceable or replaced by cations,
in that it has the X-ray powder diffraction spectrum, the most meaningful lines of which are:

| nm | $d_{\AA}$ | $I_{rel}$ |
|---|---|---|
| 1,114 ± 0,010 | (11.14 ± 0,10) | vs |
| 0,999 ± 0,010 | (9.99 ± 0,10) | s |
| 0,974 ± 0,010 | (9.74 ± 0,10) | m |
| 0,636 ± 0,007 | (6.36 ± 0,07) | mw |
| 0,599 ± 0,007 | (5.99 ± 0,07) | mw |
| 0,426 ± 0,005 | (4.26 ± 0,05) | mw |
| 0,386 ± 0,004 | (3.86 ± 0,04) | s |
| 0,382 ± 0,004 | (3.82 ± 0,04) | s |
| 0,375 ± 0,004 | (3.75 ± 0,04) | s |
| 0,372 ± 0,004 | (3.72 ± 0,04) | s |
| 0,365 ± 0,004 | (3.65 ± 0,04) | m |
| 0,365 ± 0,002 | (3.05 ± 0,02) | mw |
| 0,299 ± 0,002 | (2.99 ± 0,02) | mw |

wherein d are the interplanar distances in nm (Å) and $I_{rel}$ are their relative intensities, wherein vs means very strong; s means strong; a means medium; mw means medium-weak; w means weak,
and that it shows an I.R. spectrum having at least the following bands:

| wn | $I_{rel}$ |
|---|---|
| 1220 - 1230 | w |
| 1080 - 1110 | s |
| 965 - 975 | mw |
| 795 - 805 | mw |
| 550 - 560 | m |
| 450 - 470 | ms |

wherein wn in the wave number in $cm^{-1}$ and $I_{rel}$ are the relative intensities, wherein s means strong; ms means medium-strong; m means medium; mw means medium-weak; w means weak.

**2.** Process for the preparation of the synthetic, crystalline, porous material according to claim 1, characterized in that under hydrothermal conditions a derivative of silicon, a derivative of titanium, a derivative of iron and a nitrogenous organic base are reacted, possibly in the presence of one or more salt(s) and/or hydroxide(s) of alkali or alkali-earth metal, with an $SiO_2/Fe_2O_3$ molar ratio of the reactants greater than 50,
an $SiO_2/TiO_2$ molar ratio of the reactants greater than 5, and an $M/SiO_2$ molar ratio (wherein M is the alkali or alkali-earth cation) of the reactants lower than 0.1, or equal to zero.

**3.** Process according to claim 2, wherein the $H_2O/SiO_2$ of the reactants is comprised within the ratio of from 10 to 100.

**4.** Process according to claims 2 and 3 wherein the $SiO_2/Fe_2O_3$ molar ratio of the reactants is comprised between 150 and 600, the $SiO_2/TiO_2$ molar ratio of the reactants is comprised between 15 and 25, the $H_2O/SiO_2$ molar ratio of the reactants in comprised between 30 and 50 and the $M/SiO_2$ molar ratio of

the reactants is zero.

5. Process according to claim 2, wherein the silicon derivative is selected from silica gel, silica sol and alkyl silicates, the titanium derivative is selected from the salts and the organic derivatives of titanium, the iron derivative is selected from the salts, the hydroxides and the organic derivatives of iron.

6. Process according to claim 5, wherein the alkyl silicate is tetraethyl silicate.

7. Process according to claim 5, wherein the titanium salts are halides.

8. Process according to claim 5, wherein the organic derivatives of titanium are alkyl titanates.

9. Process according to claim 8, wherein the alkyl titanate is tetraethyltitanate.

10. Process according to claim 5, wherein the iron salts are selected from halides and nitrates.

11. Process according to claim 5, wherein the organic derivatives of iron are the alkoxides.

12. Process according to claim 2, wherein the nitrogenous base is an alkylammonium hydroxide.

13. Process according to claim 12, wherein the alkylammonium hydroxide is tetrapropylammonium hydroxide ($TPA^+$).

14. Process according to claim 2, wherein the reactants are reacted by operating at a temperature comprised within the range of from 120°C to 200°C, at a pH comprised within the range of from 9 to 14 and for a time period ranging from 1 hour to 5 days.

15. Process according to claim 14, wherein the reactants are reacted by operating at a temperature comprised within the range of from 160° to 180°C, at a pH comprised within the range of from 10 to 12 and for a time ranging from 3 hours to 10 hours.

16. Process according to claim 13, wherein the $TPA^+/SiO_2$ molar ratio of the reactants is comprised within the range of from 0,1 to 1.

17. Process according to claims 4, 5, 6, 9, 13, 15 and 16, wherein the $TPA^+/SiO_2$ molar ratio of the reactants is comprised within the range of from 0,2 to 0,4.

**Claims for the following Contracting States : AT, ES**

1. Process for preparing a synthetic, crystalline, porous material of zeolitic character, containing silicon oxide, titanium oxide and iron oxide, having,
in its calcined and anhydrous state, the following empirical formula:

$$p \ HFeO_2 \ . \ q \ TiO_2 \ . \ SiO_2$$

wherein p has a value higher than zero and lower than or equal to 0,050 and q has a value higher than zero and lower than or equal to 0,025, the $H^+$ of $HFeO_2$ can be at least partly replaceable or replaced by cations,
in that it has the X-ray powder diffraction spectrum, the most meaningful lines of which are:

EP 0 226 258 B1

| nm | $\underline{d}_{\text{Å}}$ | $I_{rel}$ |
|---|---|---|
| 1,114 ± 0,010 | (11.14 ± 0,10] | vs |
| 0,999 ± 0,010 | (9.99 ± 0,10) | s |
| 0,974 ± 0,010 | (9.74 ± 0,10) | m |
| 0,636 ± 0,007 | (6.36 ± 0,07) | mw |
| 0,599 ± 0,007 | (5.99 ± 0,07) | mw |
| 0,426 ± 0,005 | (4.26 ± 0,05) | mw |
| 0,386 ± 0,004 | (3.86 ± 0,04) | s |
| 0,382 ± 0,004 | (3.82 ± 0,04) | s |
| 0,375 ± 0,004 | (3.75 ± 0,04) | s |
| 0,372 ± 0,004 | (3.72 ± 0,04) | s |
| 0,365 ± 0,004 | (3.65 ± 0,04) | m |
| 0,365 ± 0,002 | (5.05 ± 0,02) | mw |
| 0,299 ± 0,002 | (2.99 ± 0,02) | mw |

wherein $\underline{d}$ are the interplanar distances in nm (Å) and $I_{rel}$ are their relative intensities, wherein vs means very strong; s means strong; m means medium; mw means medium-weak; w means weak, and that it shows an I.R. spectrum having at least the following bands:

| wn | $I_{rel}$ |
|---|---|
| 1220 - 1230 | w |
| 1080 - 1110 | s |
| 965 - 975 | mw |
| 795 - 805 | mw |
| 550 - 560 | m |
| 450 - 470 | ms |

wherein wn in the wave number in $cm^{-1}$ and $I_{rel}$ are the relative intensities, wherein s means strong; ms means medium-strong; m means medium; mw means medium-weak; w means weak, characterized in that, under hydrothermal conditions a derivative of silicon, a derivative of titanium, a derivative of iron and a nitrogenous organic base are reacted, possibly in the presence of one or more salt(s) and/or hydroxide(s) of alkali or alkali-earth metal, with an $SiO_2/Fe_2O_3$ molar ratio of the reactants greater than 50, an $SiO_2/TiO_2$ molar ratio of the reactants greater than 5, and an $M/SiO_2$ molar ratio (wherein M is the alkali or alkali-earth cation) of the reactants lower than 0.1, or equal to zero.

2. Process according to claim 1, wherein the $H_2O/SiO_2$ of the reactants is comprised within the ratio of from 10 to 100.

3. Process according to claims 1 and 2 wherein the $SiO_2/Fe_2O_3$ molar ratio of the reactants is comprised between 150 and 600, the $SiO_2/TiO_2$ molar ratio of the reactants is comprised between 15 and 25, the $H_2O/SiO_2$ molar ratio of the reactants in comprised between 30 and 50 and the $M/SiO_2$ molar ratio of the reactants is zero.

4. Process according to claim 1, wherein the silicon derivative is selected from silica gel, silica sol and alkyl silicates, the titanium derivative is selected from the salts and the organic derivatives of titanium, the iron derivative is selected from the salts, the hydroxides and the organic derivatives of iron.

5. Process according to claim 4, wherein the alkyl silicate is tetraethyl silicate.

6. Process according to claim 4, wherein the titanium salts are halides.

7. Process according to claim 4, wherein the organic derivatives of titanium are alkyl titanates.

8. Process according to claim 7, wherein the alkyl titanate is tetraethyltitanate.

10

**9.** Process according to claim 4, wherein the iron salts are selected from halides and nitrates.

**10.** Process according to claim 4, wherein the organic derivatives of iron are the alkoxides.

**11.** Process according to claim 1, wherein the nitrogenous base is an alkylammonium hydroxide.

**12.** Process according to claim 11, wherein the alkylammonium hydroxide is tetrapropylammonium hydroxide ($TPA^+$).

**13.** Process according to claim 1, wherein the reactants are reacted by operating at a temperature comprised within the range of from 120°C to 200°C, at a pH comprised within the range of from 9 to 14 and for a time period ranging from 1 hour to 5 days.

**14.** Process according to claim 13, wherein the reactants are reacted by operating at a temperature comprised within the range of from 160°C to 180°C, at a pH comprised within the range of from 10 to 12 and for a time ranging from 3 hours to 10 hours.

**15.** Process according to claim 14, wherein the $TPA^+/SiO_2$ molar ratio of the reactants is comprised within the range of from 0,1 to 1.

**16.** Process according to claims 3, 4, 5, 8, 13, 14 and 15, wherein the $TPA^+/SiO_2$ molar ratio of the reactants is comprised within the range of from 0,2 to 0,4.

**Revendications**

**Revendications pour les Etats contractants suivants : BE, CH, DE, FR, GB, GR, LI, LU, NL, SE**

**1.** Matériau synthétique, cristallin et poreux, de type zéolithe, contenant de l'oxyde de silicium, de l'oxyde de titane et de l'oxyde de fer, **caractérisé** en ce qu'il correspond, à l'état calciné et anhydre, à la formule empirique suivante :

p $HFeO_2$.q $TiO_2$.$SiO_2$

dans laquelle p a une valeur supérieure à zéro et inférieure ou égale à 0,050 et q a une valeur supérieure à zéro et inférieure ou égale à 0,025, l'ion $H^+$ de $HFeO_2$ pouvant être au moins partiellement remplaçable ou remplacé par des cations,
en ce qu'il présente, à l'état de poudre, un spectre de diffraction des rayons X dont les raies les plus significatives sont les suivantes :

| nm | $d_{\overset{\circ}{A}}$ | $I_{rel}$ |
|---|---|---|
| 1,114 ± 0,010 | (11,14 ± 0,10) | TF |
| 0,999 ± 0,010 | (9,99 ± 0,10) | F |
| 0,974 ± 0,010 | (9,74 ± 0,10) | m |
| 0,636 ± 0,007 | (6,36 ± 0,07) | mf |
| 0,599 ± 0,007 | (5,99 ± 0,07) | mf |
| 0,426 ± 0,005 | (4,26 ± 0,05) | mf |
| 0,386 ± 0,004 | (3,86 ± 0,04) | F |
| 0,382 ± 0 004 | (3,82 ± 0,04) | F |
| 0,375 ± 0,004 | (3,75 ± 0,04) | F |
| 0,372 ± 0,004 | (3,72 ± 0,04) | F |
| 0,365 ± 0,004 | (3,65 ± 0,04) | m |
| 0,305 ± 0,002 | (3,05 ± 0,02) | mf |
| 0,299 ± 0,002 | (2,99 ± 0,02) | mf |

où d représente les distances interplanaires en nm (Å) et $I_{rel}$ représente l'intensité relative, TF signifiant "très forte", F, "forte", m, "moyenne", mf, "moyenne-faible", et f, "faible",
et en ce qu'il présente un spectre IR dans lequel on trouve au moins les bandes suivantes :

| NO | $I_{rel}$ |
|---|---|
| 1220 - 1230 | f |
| 1080 - 1110 | F |
| 965 - 975 | mf |
| 795 - 805 | mf |
| 550 - 560 | m |
| 450 - 470 | mF |

où NO représente le nombre d'ondes en $cm^{-1}$ et $I_{rel}$ représente l'intensité relative, F signifiant "forte", mF, "moyenne-forte", m, "moyenne", mf, "moyenne-faible", et f, "faible".

2. Procédé de préparation du matériau synthétique, poreux et cristallin, conforme à la revendication 1, **caractérisé** en ce que l'on fait réagir, dans des conditions hydrothermiques, un dérivé du silicium, un dérivé du titane, un dérivé du fer et une base organique azotée, éventuellement en présence d'un ou plusieurs sel(s) et/ou hydroxyde(s) de métal alcalin ou alcalino-terreux, le rapport molaire des réactifs $SiO_2/Fe_2O_3$ valant plus de 50, le rapport molaire des réactifs $SiO_2/TiO_2$ valant plus de 5 et le rapport molaire des réactifs $M/SiO_2$ (où M représente le cation alcalin ou alcalino-terreux) étant nul ou inférieur à 0,1.

3. Procédé conforme à la revendication 2, dans lequel le rapport de réactifs $H_2O/SiO_2$ vaut de 10 à 100.

4. Procédé conforme aux revendications 2 et 3, dans lequel le rapport molaire des réactifs $SiO_2/Fe_2O_3$ vaut entre 150 et 600, le rapport molaire des réactifs $SiO_2/TiO_2$ vaut entre 15 et 25, le rapport molaire des réactifs $H_2O/SiO_2$ vaut entre 30 et 50 et le rapport molaire des réactifs $M/SiO_2$ est nul.

5. Procédé conforme à la revendication 2, dans lequel le dérivé du silicium est choisi parmi un gel de silice, un sol de silice et des silicates d'alkyle, le dérivé du titane est choisi parmi les sels et les dérivés organiques du titane, et le dérivé du fer est choisi parmi les sels, les hydroxydes et les dérivés organiques du fer.

6. Procédé conforme à la revendication 5, dans lequel le silicate d'alkyle est du silicate de tétraéthyle.

7. Procédé conforme à la revendication 5, dans lequel les sels de titane sont des halogénures.

8. Procédé conforme à la revendication 5, dans lequel les dérivés organiques du titane sont des titanates d'alkyle.

9. Procédé conforme à la revendication 8, dans lequel le titanate d'alkyle est du titanate de tétraéthyle.

10. Procédé conforme à la revendication 5, dans lequel les sels de fer sont choisis parmi les halogénures et les nitrates.

11. Procédé conforme à la revendication 5, dans lequel les dérivés organiques du fer sont des alcoxydes.

12. Procédé conforme à la revendication 2, dans lequel la base azotée est un hydroxyde d'alkyl-ammonium.

13. Procédé conforme à la revendication 12, dans lequel l'hydroxyde d'alkyl-ammonium est l'hydroxyde de tétrapropyl-ammonium ($TPA^+$).

14. Procédé conforme à la revendication 2, dans lequel on fait réagir les réactifs à une température comprise dans l'intervalle allant de 120°C à 200°C, à un pH situé dans l'intervalle allant de 9 à 14 et pendant un laps de temps valant de 1 heure à 5 jours.

15. Procédé conforme à la revendication 14, dans lequel on fait réagir les réactifs à une température située dans l'intervalle allant de 160°C à 180°C, à un pH situé dans l'intervalle allant de 10 à 12 et pendant un laps de temps valant de 3 à 10 heures.

12

**16.** Procédé conforme à la revendication 13, dans lequel le rapport molaire des réactifs $TPA^+/SiO_2$ se situe dans l'intervalle allant de 0,1 à 1.

**17.** Procédé conforme aux revendications 4, 5, 6, 9, 13, 15 et 16, dans lequel le rapport molaire des réactifs $TPA^+/SiO_2$ se situe dans l'intervalle allant de 0,2 à 0,4.

**Revendications pour les Etats contractants suivants : AT, ES**

**1.** Procédé de préparation d'un matériau synthétique, cristallin et poreux, de type zéolithe, contenant de l'oxyde de silicium, de l'oxyde de titane et de l'oxyde de fer,
correspondant, à l'état calciné et anhydre, à la formule empirique suivante :

$$p \ HFeO_2.q \ TiO_2.SiO_2$$

dans laquelle p a une valeur supérieure à zéro et inférieure ou égale à 0,050 et q a une valeur supérieure à zéro et inférieure ou égale à 0,025, l'ion $H^+$ de $HFeO_2$ pouvant être au moins partiellement remplaçable ou remplacé par des cations,
présentant, à l'état de poudre, un spectre de diffraction des rayons X dont les raies les plus significatives sont les suivantes :

| nm | $\underline{d}_Å$ | $I_{rel}$ |
|---|---|---|
| 1,114 ± 0,010 | (11,14 ± 0,10) | TF |
| 0,999 ± 0,010 | (9,99 ± 0,10) | F |
| 0,974 ± 0,010 | (9,74 ± 0,10) | m |
| 0,636 ± 0,007 | (6,36 ± 0,07) | mf |
| 0,599 ± 0,007 | (5,99 ± 0,07) | mf |
| 0,426 ± 0,005 | (4,26 ± 0,05) | mf |
| 0,386 ± 0,004 | (3,86 ± 0,04) | F |
| 0,382 ± 0,004 | (3,82 ± 0,04) | F |
| 0,375 ± 0,004 | (3,75 ± 0,04) | F |
| 0,372 ± 0,004 | (3,72 ± 0,04) | F |
| 0,365 ± 0 004 | (3,65 ± 0,04) | m |
| 0,305 ± 0,002 | (3,05 ± 0,02) | mf |
| 0,299 ± 0 002 | (2,99 ± 0,02) | mf |

où d représente les distances interplanaires en nm (Å) et $I_{rel}$ représente l'intensité relative, TF signifiant "très forte", F, "forte", m, "moyenne", mf, "moyenne-faible", et f, "faible",
présentant un spectre IR dans lequel on trouve au moins les bandes suivantes :

| NO | $I_{rel}$ |
|---|---|
| 1220 - 1230 | f |
| 1080 - 1110 | F |
| 965 - 975 | mf |
| 795 - 805 | mf |
| 550 - 560 | m |
| 450 - 470 | mF |

où NO représente le nombre d'ondes en $cm^{-1}$ et $I_{rel}$ représente l'intensité relative, F signifiant "forte", mF, "moyenne-forte", m, "moyenne", mf, "moyenne-faible", et f, "faible",
**caractérisé** en ce que l'on fait réagir, dans des conditions hydrothermiques, un dérivé du silicium, un dérivé du titane, un dérivé du fer et une base organique azotée, éventuellement en présence d'un ou plusieurs sel(s) et/ou hydroxyde(s) de métal alcalin ou alcalino-terreux, le rapport molaire des réactifs $SiO_2/Fe_2O_3$ valant plus de 50, le rapport molaire des réactifs $SiO_2/TiO_2$ valant plus de 5 et le rapport molaire des réactifs $M/SiO_2$ (où M représente le cation alcalin ou alcalino-terreux) étant nul ou inférieur à 0,1.

**2.** Procédé conforme à la revendication 1 dans lequel le rapport de réactifs $H_2O/SiO_2$ vaut de 10 à 100.

**3.** Procédé conforme aux revendications 1 et 2, dans lequel le rapport molaire des réactifs $SiO_2/Fe_2O_3$ vaut entre 150 et 600, le rapport molaire des réactifs $SiO_2/TiO_2$ vaut entre 15 et 25, le rapport molaire des réactifs $H_2O/SiO_2$ vaut entre 30 et 50 et le rapport molaire des réactifs $M/SiO_2$ est nul.

**4.** Procédé conforme à la revendication 1, dans lequel le dérivé du silicium est choisi parmi un gel de silice, un sol de silice et des silicates d'alkyle, le dérivé du titane est choisi parmi les sels et les dérivés organiques du titane, et le dérivé du fer est choisi parmi les sels, les hydroxydes et les dérivés organiques du fer.

**5.** Procédé conforme à la revendication 4, dans lequel le silicate d'alkyle est du silicate de tétraéthyle.

**6.** Procédé conforme à la revendication 4, dans lequel les sels de titane sont des halogénures.

**7.** Procédé conforme à la revendication 4, dans lequel les dérivés organiques du titane sont des titanates d'alkyle.

**8.** Procédé conforme à la revendication 7, dans lequel le titanate d'alkyle est du titanate de tétraéthyle.

**9.** Procédé conforme à la revendication 4, dans lequel les sels de fer sont choisis parmi les halogénures et les nitrates.

**10.** Procédé conforme à la revendication 4, dans lequel les dérivés organiques du fer sont des alcoxydes.

**11.** Procédé conforme à la revendication 1, dans lequel la base azotée est un hydroxyde d'alkyl-ammonium.

**12.** Procédé conforme à la revendication 11, dans lequel l'hydroxyde d'alkyl-ammonium est l'hydroxyde de tétrapropyl-ammonium ($TPA^+$).

**13.** Procédé conforme à la revendication 1, dans lequel on fait réagir les réactifs à une température comprise dans l'intervalle allant de 120°C à 200°C, à un pH situé dans l'intervalle allant de 9 à 14 et pendant un laps de temps valant de 1 heure à 5 jours.

**14.** Procédé conforme à la revendication 13, dans lequel on fait réagir les réactifs à une température située dans l'intervalle allant de 160°C à 180°C, à un pH situé dans l'intervalle allant de 10 à 12 et pendant un laps de temps valant de 3 à 10 heures.

**15.** Procédé conforme à la revendication 14, dans lequel le rapport molaire des réactifs $TPA^+/SiO_2$ se situe dans l'intervalle allant de 0,1 à 1.

**16.** Procédé conforme aux revendications 3, 4, 5, 8, 13, 14 et 15, dans lequel le rapport molaire des réactifs $TPA^+/SiO_2$ se situe dans l'intervalle allant de 0,2 à 0,4.

**Patentansprüche**
**Patentansprüche für folgende Vertragsstaaten : BE, CH, DE, FR, GB, GR, LI, LU, NL, SE**

**1.** Synthetisches, kristallines, poröses Material mit Zeolithcharakter und einem Gehalt an Siliziumoxid, Titanoxid und Eisenoxid, dadurch gekennzeichnet, daß es in seinem calcinierten und wasserfreien Zustand der folgenden empirischen Formel:

$$p\ HFeO_2\ .\ q\ TiO_2\ .\ SiO_2$$

entspricht, worin p einen Wert größer als null und kleiner als oder gleich 0,050 aufweist und q einen Wert größer als null und kleiner als oder gleich 0,025 hat, wobei $H^+$ von $HFeO_2$ wenigstens teilweise durch Kationen ersetzbar oder ersetzt ist,
daß in seinem Röntgen-Pulverbeugungsspektrum die wichtigsten Linien wie folgt sind:

EP 0 226 258 B1

| nm | $d_\text{Å}$ | $I_\text{rel}$ |
|---|---|---|
| 1,114 ± 0,010 | (11,14 ± 0,10) | vs |
| 0,999 ± 0,010 | (9,99 ± 0,10) | s |
| 0,974 ± 0,010 | (9,74 ± 0,10) | m |
| 0,636 ± 0,007 | (6,36 ± 0,07) | mw |
| 0,599 ± 0,007 | (5,99 ± 0,07) | mw |
| 0,426 ± 0,005 | (4,26 ± 0,05) | mw |
| 0,386 ± 0,004 | (3,86 ± 0,04) | s |
| 0,382 ± 0,004 | (3,82 ± 0,04) | s |
| 0,375 ± 0,004 | (3,75 ± 0,04) | s |
| 0,372 ± 0,004 | (3,72 ± 0,04) | s |
| 0,365 ± 0,004 | (3,65 ± 0,04) | m |
| 0,305 ± 0,002 | (3,05 ± 0,02) | mw |
| 0,299 ± 0,002 | (2,99 ± 0,02) | mw, |

worin d die Netzebenenabstände in nm (Å) und $I_\text{rel}$ die relativen Inensitäten bezeichnen, und vs sehr stark; s stark; m mittel; mw mittelschwach; w schwach bedeuten,
und daß es ein Infrarotspektrum mit wenigstens den folgenden Banden zeigt:

| wn | $I_\text{rel}$ |
|---|---|
| 1.220 - 1.230 | w |
| 1.080 - 1.110 | s |
| 965 - 975 | mw |
| 795 - 805 | mw |
| 550 - 560 | m |
| 450 - 470 | ms, |

worin wn die Wellenzahl in $cm^{-1}$ darstellt und $I_\text{rel}$ die relativen Intensitäten angibt, worin s stark; ms mittelstark; m mittel; mw mittelwschwach; und w schwach bedeuten.

2. Verfahren zur Herstellung des synthetischen, kristallinen, porösen Materials nach Anspruch 1, dadurch gekennzeichnet, daß unter hydrothermalen Bedingungen ein Derivat von Silizium, ein Derivat von Titan, ein Derivat von Eisen und eine stickstoffhaltige organische Base, gegebenenfalls in Anwesenheit eines oder mehrerer Salze und/oder Hydroxide von Alkali- oder Erdalkalimetallen, bei einem $SiO_2/Fe_2O_3$ - Molverhältnis der Reaktantan von über 50, einem $SiO_2/TiO_2$-Molverhältnis der Reaktanten von über 5 und einem $M/SiO_2$-Molverhältnis (worin M das Alkali- oder Erdalkalimetallkation ist) der Reaktanten von unter 0,1 oder gleich null umgesetzt werden.

3. Verfahren nach Anspruch 2, worin das $H_2O/SiO_2$-Verhältnis der Reaktanten im Bereich von 10 bis 100 liegt.

4. Verfahren nach Anspruch 2 und 3, worin das $SiO_2/Fe_2O_3$-Molverhältnis der Reaktanten von 150 bis 600 beträgt, das $SiO_2/TiO_2$-Molverhältnis der Reaktanten von 15 bis 25 beträgt, das $H_2O/SiO_2$-Molverhältnis der Reaktanten 30 bis 50 beträgt und das $M/SiO_2$-Molverhältnis der Reaktanten null ausmacht.

5. Verfahren nach Anspruch 2, worin das Siliziumderivat unter Silikagel, Silikasol und Alkylsilikaten ausgewählt ist, das Titanderivat unter den Salzen und organischen Derivaten von Titan ausgewählt ist und das Eisenderivat unter den Salzen, Hydroxiden und organischen Derivaten von Eisen ausgewählt ist.

6. Verfahren nach Anspruch 5, worin das Alkylsilikat Tetraethylsilikat ist.

7. Verfahren nach Anspruch 5, worin die Titansalze Halogenide sind.

8. Verfahren nach Anspruch 5, worin die organischen Derivate von Titan Alkyltitanate sind.

15

**9.** Verfahren nach Anspruch 8, worin das Alkyltitanat Tetraethyltitanat ist.

**10.** Verfahren nach Anspruch 5, worin die Eisensalze unter Halogeniden und Nitraten ausgewählt sind.

**11.** Verfahren nach Anspruch 5, worin die organischen Derivate von Eisen die Alkoxide sind.

**12.** Verfahren nach Anspruch 2, worin die stickstoffhaltige Base ein Alkylammoniumhydroxid ist.

**13.** Verfahren nach Anspruch 12, worin das Alkylammoniumhydroxid Tetrapropylammoniumhydroxid ($TPA^+$) ist.

**14.** Verfahren nach Anspruch 2, worin die Reaktanten durch Arbeiten bei einer Temperatur im Bereich von 120°C bis 200°C, bei einem pH-Wert im Bereich von 9 bis 14 und während einer Zeitdauer von einer Stunde bis fünf Tagen zur Reaktion gebracht werden.

**15.** Verfahren nach Anspruch 14, worin die Reaktanten durch Arbeiten bei einer Temperatur im Bereich von 160°C bis 180°C, bei einem pH-Wert im Bereich von 10 bis 12 und während einer Zeitdauer von 3 Stunden bis 10 Stunden miteinander zur Reaktion gebracht werden.

**16.** Verfahren nach Anspruch 13, worin das $TPA^+/SiO_2$-Molverhältnis der Reaktanten im Bereich von 0,1 bis 1 liegt.

**17.** Verfahren nach den Ansprüchen 4, 5, 6, 9, 13, 15 und 16, worin das $TPA^+/SiO_2$-Molverhältnis der Reaktanten im Bereich von 0,2 bis 0,4 liegt.

**Patentansprüche für folgende Vertragsstaaten : AT, ES**

**1.** Verfahren zur Herstellung eines synthetischen, kristallinen, porösen Materials von Zeolithcharakter mit einem Gehalt an Siliziumoxid, Titanoxid und Eisenoxid, das in seiner calcinierten und wasserfreien Form die folgende empirische Formel aufweist:

$$p\ HFeO_2\ .\ q\ TiO_2\ .\ SiO_2,$$

worin p einen Wert größer als null und kleiner als oder gleich 0,050 aufweist und q einen Wert größer als null und kleiner als oder gleich 0,025 hat, wobei $H^+$ von $HFeO_2$ wenigstens teilweise durch Kationen ersetzbar oder ersetzt ist,
das in seinem Röntgen-Pulverbeugungsspektrum die wichtigsten Linien wie folgt zeigt:

| nm | $d_Å$ | $I_{rel}$ |
|---|---|---|
| 1,114 ± 0,010 | (11,14 ± 0,10) | vs |
| 0,999 ± 0,010 | (9,99 ± 0,10) | s |
| 0,974 ± 0,010 | (9,74 ± 0,10) | m |
| 0,636 ± 0,007 | (6,36 ± 0,07) | mw |
| 0,599 ± 0,007 | (5,99 ± 0,07) | mw |
| 0,426 ± 0,005 | (4,26 ± 0,05) | mw |
| 0,386 ± 0,004 | (3,86 ± 0,04) | s |
| 0,382 ± 0,004 | (3,82 ± 0,04) | s |
| 0,375 ± 0,004 | (3,75 ± 0,04) | s |
| 0,372 ± 0,004 | (3,72 ± 0,04) | s |
| 0,365 ± 0,004 | (3,65 ± 0,04) | m |
| 0,305 ± 0,002 | (3,05 ± 0,02) | mw |
| 0,299 ± 0,002 | (2,99 ± 0,02) | mw, |

worin d die Netzebenenabstände in nm (Å) und $I_{rel}$ die relativen Inensitäten bezeichnen, und vs sehr stark; s stark; m mittel; mw mittelschwach; w schwach bedeuten,
und das ein Infrarotspektrum mit wenigstens den folgenden Banden zeigt:

EP 0 226 258 B1

| wn | $I_{rel}$ |
|---|---|
| 1.220 - 1.230 | w |
| 1.080 - 1.110 | s |
| 965 - 975 | mw |
| 795 805 | mw |
| 550 - 560 | m |
| 450 - 470 | ms, |

worin wn die Wellenzahl in $cm^{-1}$ darstellt und $I_{rel}$ die relativen Intensitäten angibt, worin s stark; ms mittelstark; m mittel; mw mittelwschwach; und w schwach bedeuten,
dadurch gekennzeichnet, daß unter hydrothermalen Bedingungen ein Derivat von Silizium, ein Derivat von Titan, ein Derivat von Eisen und eine stickstoffhaltige organische Base, gegebenenfalls in Anwesenheit eines oder mehrerer Salze und/oder Hydroxide von Alkali- oder Erdalkalimetallen, bei einem $SiO_2/Fe_2O_3$-Molverhältnis der Reaktanten von über 50, einem $SiO_2/TiO_2$-Molverhältnis der Reaktanten von über 5 und einem $M/SiO_2$-Molverhältnis (worin M das Alkali- oder Erdalkalimetallkation ist) der Reaktanten von unter 0,1 oder gleich null umgesetzt werden.

2. Verfahren nach Anspruch 1, worin das $H_2O/SiO_2$-Verhältnis der Reaktanten im Bereich von 10 bis 100 liegt.

3. Verfahren nach Anspruch 1 und 2, worin das $SiO_2/Fe_2O_3$-Molverhältnis der Reaktanten von 150 bis 600 beträgt, das $SiO_2/TiO_2$-Molverhältnis der Reaktanten von 15 bis 25 beträgt, das $H_2O/SiO_2$-Molverhältnis der Reaktanten 30 bis 50 beträgt und das $M/SiO_2$-Molverhältnis der Reaktanten null ausmacht.

4. Verfahren nach Anspruch 1, worin das Siliziumderivat unter Silikagel, Silikasol und Alkylsilikaten ausgewählt ist, das Titanderivat unter den Salzen und organischen Derivaten von Titan ausgewählt ist und das Eisenderivat unter den Salzen, Hydroxiden und organischen Derivaten von Eisen ausgewählt ist.

5. Verfahren nach Anspruch 4, worin das Alkylsilikat Tetraethylsilikat ist.

6. Verfahren nach Anspruch 4, worin die Titansalze Halogenide sind.

7. Verfahren nach Anspruch 4, worin die organischen Derivate von Titan Alkyltitanate sind.

8. Verfahren nach Anspruch 7, worin das Alkyltitanat Tetraethyltitanat ist.

9. Verfahren nach Anspruch 4, worin die Eisensalze unter Halogeniden und Nitraten ausgewählt sind.

10. Verfahren nach Anspruch 4, worin die organischen Derivate von Eisen die Alkoxide sind.

11. Verfahren nach Anspruch 1, worin die stickstoffhaltige Base ein Alkylammoniumhydroxid ist.

12. Verfahren nach Anspruch 11, worin das Alkylammoniumhydroxid Tetrapropylammoniumhydroxid $(TPA^+)$ ist.

13. Verfahren nach Anspruch 1, worin die Reaktanten durch Arbeiten bei einer Temperatur im Bereich von $120\,°C$ bis $200\,°C$, bei einem pH-Wert im Bereich von 9 bis 14 und während einer Zeitdauer von einer Stunde bis fünf Tagen zur Reaktion gebracht werden.

14. Verfahren nach Anspruch 13, worin die Reaktanten durch Arbeiten bei einer Temperatur im Bereich von $160\,°C$ bis $180\,°C$, bei einem pH-Wert im Bereich von 10 bis 12 und während einer Zeitdauer von 3 Stunden bis 10 Stunden miteinander zur Reaktion gebracht werden.

15. Verfahren nach Anspruch 14, worin das $TPA^+/SiO_2$-Molverhältnis der Reaktanten im Bereich von 0,1 bis 1 liegt.

17

**16.** Verfahren nach den Ansprüchen 3, 4, 5, 8, 13, 14 und 15, worin das $TPA^+/SiO_2$-Molverhältnis der Reaktanten im Bereich von 0,2 bis 0,4 liegt.

## Fig.1

## Fig.2

## Fig.3

## Fig.4